# FASCICULE DE BREVET EUROPEEN

(11) **EP 3 047 281 B1**
(45) Date de publication et mention de la délivrance du brevet: **09.01.2019**
(21) Numéro de dépôt: 14793211.5
(22) Date de dépôt: 18.09.2014
(51) Int. Cl.: G01N 33/68

(54) **PROCÉDE D'ÉVALUATION DU RISQUE DE MORTALITÉ CHEZ DES PATIENTS QUI PRÉSENTENT UN SYNDROME DE RÉPONSE INFLAMMATOIRE SYSTÉMIQUE (SIRS) OU UN SEPSIS**
VERFAHREN ZUR BEURTEILUNG DES STERBLICHKEITSRISIKOS BEI PATIENTEN MIT SYSTEMISCHEM INFLAMMATORISCHEM RESPONSE-SYNDROM (SIRS) ODER SEPSIS
METHOD FOR EVALUATING THE RISK OF MORTALITY IN PATIENTS HAVING SYSTEMIC INFLAMMATORY RESPONSE SYNDROME (SIRS) OR SEPTIC SHOCK

(30) Priorité: 18.09.2013 FR 1358979
(43) Date de publication de la demande: 27.07.2016
(73) Titulaire: Biomérieux, 69280 Marcy l'Étoile (FR); Hospices Civils de Lyon, 69229 Lyon Cedex 02 (FR)
(72) Inventeur: MONNERET, Guillaume, F-69003 Lyon (FR); VENET, Fabienne, F-69003 Lyon (FR); LEPAPE, Alain, F-69230 Saint-Genis Laval (FR); DEMARET, Julie, F-69006 Lyon (FR); VILLARS-MECHIN, Astrid, 69680 Chassieu (FR)
(74) Mandataire: Sarlin, Laure V.
(86) Numéro de dépôt international: PCT/FR2014/052318
(87) Numéro de publication internationale: WO 2015/040328

(56) Documents cités:
- WO-A1-2012/096245
- VENET F. ET AL.: "IL-7 restores lymphocyte functions in septic patients", J. IMMUNOL., vol. 189, no. 10, 15 novembre 2012 (2012-11-15), pages 5073-5081, XP055100771,
- UNSINGER J. ET AL.: "IL-7 promotes T cell viability, trafficking, and functionality and improves survival in sepsis", J. IMMUNOL., vol. 184, no. 7, mars 2010 (2010-03), pages 3768-3779, XP055100934,
- FAUCHER S. ET AL.: "Development of a quantitative bead capture assay for soluble IL-7 receptor alpha in human plasma", PLOS ONE, vol. 4, no. 8, E6690, 19 août 2009 (2009-08-19), pages 1-6, XP055100777,
- DEMARET J. ET AL.: "Elevated plasmatic level of soluble IL-7 receptor is associated with increased mortality in septic shock patients", INTENSIVE CARE MED., vol. 40, no. 8, août 2014 (2014-08), pages 1089-1096, XP55154713,

## Description

La présente invention concerne le domaine médical en général, et en particulier le domaine de la réanimation.

Plus précisément, l'invention concerne un procédé d'évaluation du risque de mortalité chez un patient ayant subi une agression, telle que chirurgie, brûlure, traumatisme..., générant une réponse inflammatoire systémique ou SIRS ou chez un patient en état septique, c'est-à-dire un patient présentant un SEPSIS, notamment un SEPSIS sévère, également nommé SEPSIS grave, et de préférence chez un patient en choc septique.

Le sepsis est un syndrome de réponse systémique inflammatoire en relation avec une Infection.

Un sepsis sévère est un sepsis associé à une hypotension artérielle et/ou hypoperfuslon et/ou au dysfonctionnement d'au moins un organe.

Le choc septique est un sepsis sévère associé à une hypotension persistante malgré des remplissages adéquats et des traitements vasopresseurs.

La différence entre SEPSIS, SEPSIS sévère et choc septique réside principalement dans l'importance de la perturbation de toutes les fonctions vitales.

Les patients SIRS et présentant des syndromes septiques, c'est-à-dire les patients en état septique allant de SEPSIS, SEPSIS sévère, à choc septique, représentent l'une des premières causes de mortalité dans les services de réanimation.

Estimer le risque de mortalité d'un patient SIRS, SEPSIS, SEPSIS sévère et en particulier d'un patient en état de choc septique, est donc essentiel pour pouvoir proposer une prise en charge personnalisée, et ainsi tenter de réduire le risque de décès.

La sévérité de l'état d'un patient admis en service de réanimation est généralement estimée à l'aide de différents paramètres cliniques et physiologiques. Ceux-ci permettent notamment de définir des scores prédictifs en termes de survie/mortalité, parmi lesquels on peut notamment citer les scores de sévérité SOFA (*Sequential Organ Failure Assessment* ou *Sepsis-related Organ Failure Assessment*) et SAPSII (*Simplified Acute Physiology Score II*) / IGS II (Indice de Gravité Simplifié II). Ces scores composites, définis sur des cohortes conséquentes de patients de réanimation, incluent plusieurs paramètres clinico-biologiques tels que le nombre de plaquettes circulantes, la bilirubinémie, la diurése, l'âge ou la température corporelle. Ces scores permettent d'évaluer par le calcul d'une valeur numérique le degré d'atteinte de la fonction d'un ou de plusieurs systèmes physiologiques (par exemple : cardiovasculaire, rénal, cérébral). Ils sont calculés durant les premiers jours d'admission en réanimation. Dans le cas du score SAPSII, seule la valeur la plus délétère des paramètres inclus dans le score, mesurée au cours des 24 premières heures de séjour en réanimation, est prise en compte.

Ces scores sont néanmoins peu pratiques en routine clinique car ils nécessitent, de la part du médecin, une démarche active de recherche des paramètres cliniques dans le dossier du patient.

Ainsi, il existe un réel besoin de disposer d'autres outils, et notamment de marqueurs mesurables, permettant d'évaluer facilement et rapidement le risque de mortalité d'un patient admis en service de réanimation qui est par définition dans un état grave, et chez lequel le pronostic vital peut éventuellement être rapidement engagé. En effet, pouvoir identifier les sujets dont le risque de mortalité est accru permettrait de leur réserver une prise en charge, un suivi ainsi que des thérapeutiques plus adaptés.

WO 2012/096245 A1 concerne un procédé de prédiction du pronostic d'un choc septique, en particulier, du risque de mortalité chez un patient en état de choc septique, caractérisé par la mesure de l'antigène CD14 soluble (sCD14-ST). Ce document démontre que sCD14-ST est un meilleur marqueur que la procalcitonine, connu pour la prédiction du pronostic d'un patient en état de choc septique.

C'est dans ce contexte que la présente invention se propose de fournir un nouveau « biomarqueur » prédictif d'un risque accru de mortalité chez un patient ayant subi une agression sévère (chirurgie, brûlure, traumatisme...) générant une réponse inflammatoire systémique (SIRS) ou chez un patient présentant un SEPSIS, en particulier SEPSIS sévère, et de préférence chez un patient en état de choc septique. L'étude du niveau d'expression de ce « biomarqueur » permet ainsi d'évaluer, facilement et rapidement, le risque de mortalité du patient et de prendre toutes les dispositions préventives possibles.

Selon un premier aspect, la présente invention a donc pour objet un procédé d'évaluation du risque de mortalité chez un patient ayant subi une agression, telle que chirurgie, brûlure, traumatisme..., ou une infection générant une réponse inflammatoire systémique ou SIRS, comprenant, voire consistant en les étapes suivantes :
- mesurer l'expression de sCD127 dans un échantillon biologique issu dudit patient, également nommé échantillon à tester,
- conclure à un risque accru de mortalité après comparaison de l'expression de sCD127 par rapport à une valeur de référence.

Le procédé selon l'invention est un procédé mis en oeuvre *in vitro* ou *ex vivo.* Il présente l'avantage de pouvoir évaluer facilement le risque de mortalité, notamment d'un patient qui est admis en service de réanimation ou en urgence, en disposant d'un marqueur directement mesurable, contrairement aux scores de sévérité SOFA et SAPSII par exemple, et dont la mesure peut être faite dans un laboratoire de proximité ou au lit du patient. La mesure du marqueur est tout à fait adaptée pour être réalisée par des automates d'analyse ou par des tests dits rapides.

Le sCD127 est la forme soluble ou plasmatique du CD127, récepteur de l'IL-7. Le CD127 ou chaîne alpha du récepteur de l'IL-7 est une glycoprotéine de 75 kDa membre de la superfamille des récepteurs aux facteurs de croissance hématopoïétiques. Il est exprimé au niveau membranaire en association avec le CD132 (chaîne γ_{c} commune) pour former le récepteur de l'IL-7. Ce récepteur joue un rôle important dans la différenciation, la survie et la prolifération lymphocytaire. Le CD127 est constitué d'une partie extracellulaire de 219 acides aminés (aa), d'une partie transmembranaire de 25 aa et d'une partie intracytoplasmique de 195 aa. L'existence d'une forme soluble/plasmatique, nommée sCD127, générée par épissage alternatif de l'ARNm codant le CD127 a été décrite en 1990 par Goodwin RG et al., Cell, 1990, 23, 941-951, mais sa fonction biologique reste à ce jour mal connue.

Dans le cadre de l'invention, on entend par « sCD127 » la forme soluble ou forme circulante (encore nommée forme plasmatique ou sérique) du récepteur de l'IL-7, encore nommée chaîne alpha du récepteur de l'IL-7 ou IL7R ou IL7R-ALPHA ou IL7RA ou CDW127, et en particulier telle que décrite par Goodwin et al, Cell, 1990, 23, 941-951 et dosée par Crawley et al, Journal of Immunology, 2010, 184, 4679-4687.

En particulier, les séquences nucléiques de référence pour CD127 et donc pour sCD127 selon l'invention sont, de préférence, les suivantes : Ensemble : ENSG00000168685, HPRD-ID : 00893 et Séquence nucléotidique : NM 002185.2, Vega genes : OTTHUMG00000090791.

Par ailleurs, les séquences protéiques de référence pour CD127 et donc pour sCD127 selon l'invention sont de préférence les suivantes : NP_002176 XP_942460 ; version : NP_002176.2 GI:28610151.

L'échantillon à tester dans le cadre du procédé de l'invention est un échantillon biologique provenant du patient chez qui on souhaite évaluer le risque de mortalité. En particulier, un tel échantillon biologique est choisi parmi ceux susceptibles de contenir le marqueur sCD127.

Au sens de l'invention, on entend par « réponse inflammatoire systémique » ou « SIRS », une réponse associant au moins deux des critères suivants : Température > 38 °C ou < 36 °C, Fréquence cardiaque > 90/minute, Fréquence respiratoire > 20 / minute ou paCO2 <32 mmHg, Leucocytes > 12.000/mm3 ou < 4.000/mm3 (Bone et al., Chest, 1992, 1644-1655.

La présente invention présente une application préférée chez les patients qui présentent un SEPSIS, notamment un SEPSIS sévère. De manière particulièrement préférée, le procédé selon la présente invention est tout particulièrement avantageux pour évaluer le risque de mortalité chez un patient qui est en état de choc septique. Chez ces patients en état septique (SEPSIS, SEPSIS sévère et choc septique), qui présentent une SIRS suite à une infection, l'infection peut être de diverses origines, et en particulier une infection bactérienne, virale, fongique.

Selon un premier mode de réalisation préféré, le procédé de l'invention permet de conclure à un risque accru de mortalité du patient, lorsqu'une surexpression de sCD127 est mise en évidence dans l'échantillon à tester par rapport à une première valeur de référence.

Par « surexpression », on entend une augmentation significative du niveau d'expression par rapport à une valeur de référence. L'homme du métier saura déterminer le test statistique à utiliser pour déterminer la valeur de référence avec laquelle le niveau d'expression de sCD127 doit être comparé en fonction de la comparaison à effectuer, par exemple comparaison de populations ou types d'échantillons différents, comparaison de l'évolution dans le temps d'une même population ou du même type d'échantillon, etc, et déterminer une augmentation significative du niveau d'expression de sCD127 en fonction du type d'échantillons testés (par exemple plasma, sérum ou sang), du type d'analyse immunologique pratiquée (par exemple Blot, ELISA), voire de l'appareil d'analyse utilisé, etc.

Dans ce mode de réalisation, la première valeur de référence peut correspondre au niveau d'expression de sCD127 mesuré dans un échantillon biologique issu d'un patient ayant subi une agression ou une infection générant une réponse inflammatoire systémique dont on sait qu'il a survécu, notamment d'un patient présentant un SEPSIS dont on sait qu'il a survécu, et de préférence, d'un patient en choc septique dont on sait qu'il a survécu.

Dans ce cas, cette mesure de l'expression de sCD127 qui constitue la première valeur de référence est de préférence effectuée en parallèle, c'est-à-dire en même temps que la mesure de l'expression de sCD127 qui est faite dans l'échantillon issu du patient chez qui on souhaite évaluer le risque de mortalité, bien que le prélèvement de l'échantillon de référence ait été effectué antérieurement à celui de l'échantillon à tester.

Cette première valeur de référence peut également correspondre à une valeur moyenne du niveau d'expression de sCD127 qui est mesurée sur un pool d'échantillons issu de patients ayant subi une agression, telle que chirurgie, brûlure, traumatisme..., ou une infection générant une réponse inflammatoire systémique (SIRS) dont on sait qu'ils ont survécu, notamment de patients présentant un SEPSIS dont on sait qu'ils ont survécu, et, de préférence, de patients en état de choc septique dont on sait qu'ils ont survécu. Dans ce cas, cette mesure de l'expression de sCD127 qui constitue la première valeur de référence est de préférence effectuée préalablement à la mesure de l'expression de sCD127 qui est faite dans l'échantillon issu du patient chez qui on cherche à évaluer le risque de mortalité, bien que le prélèvement des échantillons de référence destinés à être « poolés » ait été effectué antérieurement à celui de l'échantillon à tester.

Dans ce premier mode de réalisation préféré, et notamment pour évaluer le risque de mortalité d'un patient en état de choc septique, la mesure de l'expression de sCD127 dans l'échantillon à tester et le cas échéant dans l'échantillon biologique utilisé pour obtenir la première valeur de référence, est réalisée dans les 4 jours ou à 4 jours (J4) après le choc septique, de préférence dans les 3 jours ou à 3 jours (13) après le choc septique, de manière encore préférée dans les 2 jours ou à 2 jours (J2) après le choc septique, et de manière particulièrement préférée dans le jour ou à 1 jour (J1) après le choc septique. Autrement dit, cette première valeur de référence est réalisée dans les 4 jours, dans les 3 jours, dans les 2 jours, dans le jour après le choc septique ou à 4 jours, à 3 jours, à 2 jours, à 1 jour après le choc septique. Selon une mise en oeuvre particulièrement avantageuse de ce premier mode de réalisation de l'invention, la première valeur de référence est réalisée dans les 2 jours ou dans le jour, ou encore à 2 jours ou à 1 jour après le choc septique, ce qui permet de déterminer de manière très précoce le risque de mortalité du patient testé.

Selon un deuxième mode de réalisation préféré, le procédé de l'invention permet de conclure à un risque accru de mortalité du patient, lorsque l'expression de sCD127 qui est mesurée dans l'échantillon à tester n'est pas significativement diminuée par rapport à une deuxième valeur de référence. Il est à la portée de l'homme du métier de déterminer le pourcentage de diminution significatif qui dépendra du type d'échantillon à tester (par exemple plasma, sérum ou sang), du type d'analyse immunologique (par exemple Blot, ELISA) voire de l'appareil sur lequel l'analyse est effectuée, etc. De manière générale, il est conclu à un risque accru de mortalité si l'expression de sCD127 qui est mesurée dans l'échantillon à tester n'est pas diminuée de plus de 30 % par rapport à cette deuxième valeur de référence, de préférence n'est pas diminuée de plus de 25 %, et en particulier n'est pas diminuée de plus de 20 %, ou encore n'est pas diminuée de plus de 15 % ou de 10 % par rapport à cette deuxième valeur de référence.

Cette deuxième valeur de référence correspond au niveau d'expression de sCD127 mesuré dans un échantillon biologique issu du même dit patient lors d'un prélèvement antérieur, c'est-à-dire dans un échantillon biologique issu du patient chez qui on souhaite évaluer le risque de mortalité et obtenu antérieurement par rapport à l'échantillon à tester. Par « antérieur » ou « antérieurement », on entend de manière plus précoce dans le temps. De préférence, la deuxième valeur de référence correspond au niveau d'expression de sCD127 mesuré dans un échantillon biologique qui est directement antérieur par rapport à l'échantillon à tester, c'est-à-dire celui qui précède l'échantillon à tester dans l'ordre des prélèvements effectués chez le patient.

Dans ce deuxième mode de réalisation préféré, et notamment pour évaluer le risque de mortalité d'un patient en état de choc septique, la mesure de l'expression de sCD127 dans l'échantillon à tester est réalisée environ ou à 7 jours (37) après le choc septique, de préférence encore environ ou à 4 jours (J4) après le choc septique, en particulier environ 3 jours ou à 3 jours après le choc septique, et de manière encore préférée environ 2 jours ou à 2 jours (J2) après le choc septique.

Le prélèvement antérieur peut par exemple être effectué dans les ou à 48h après le choc septique et au moins 24h avant celui de l'échantillon à tester, et de préférence le prélèvement antérieur est effectué dans les ou à 48h après le choc septique et celui de l'échantillon à tester est effectué dans les 48h qui suivent le prélèvement antérieur ou à 48h après le prélèvement antérieur.

Ainsi, dans tous les cas, avant la mesure de l'expression de sCD127 proprement dite dans l'échantillon à tester, le procédé de l'invention peut comprendre l'obtention préalable de la valeur de référence, que ce soit la première valeur de référence ou la deuxième valeur de référence, à laquelle le niveau d'expression qui sera détecté dans l'échantillon à tester pourra être comparé afin de conclure à un risque accru ou non de mortalité chez le patient duquel est issu l'échantillon à tester.

C'est donc à ces valeurs de référence, que ce soit la première valeur de référence ou la deuxième valeur de référence, obtenues préalablement ou en même temps, que sera comparée la valeur de l'expression de sCD127 qui est mesurée dans l'échantillon à tester.

L'échantillon sur lequel le procédé de l'invention est mis en oeuvre, encore nommé ici échantillon à tester, peut être d'origine animale ou humaine, et de préférence humaine.

L'échantillon à tester peut être un fluide biologique, par exemple choisi parmi le sang, le sang total notamment tel que collecté de la voie veineuse, c'est-à-dire contenant les cellules blanches et rouges, les plaquettes et le plasma, le sérum, le plasma et le liquide de lavage broncho-alvéolaire.

De préférence, l'échantillon à tester issu dudit patient est un échantillon de plasma ou de sérum.

Les échantillons à partir desquels peuvent être déterminées les valeurs de référence, que ce soit la première valeur de référence ou la deuxième valeur de référence, encore nommés « échantillons de référence », peuvent être de différentes natures et notamment de nature biologique tel que mentionné ci-dessus s'agissant de l'échantillon à tester (fluides biologiques). Avantageusement, ces échantillons biologiques sont de même nature que celle de l'échantillon biologique à tester ou tout du moins d'une nature compatible pour constituer une référence quant à la détection et/ou la quantification de l'expression de sCD127.

Pour obtenir la première valeur de référence en particulier, ces échantillons sont de préférence issus de personnes ayant les mêmes caractéristiques ou une majorité de caractéristiques communes, notamment du même sexe et/ou d'un âge similaire ou identique et/ou de même origine ethnique, avec celles du sujet ou patient chez qui on souhaite évaluer le risque de mortalité. L'échantillon de référence peut également dans ce cas être constitué par tout échantillon, biologique ou non biologique, qui a été préalablement calibré pour contenir une valeur moyenne de sCD127 qui correspond au niveau qui a été mesuré sur un pool d'échantillons biologiques issus de patients ayant subi une agression, telle que chirurgie, brûlure, traumatisme..., ou une infection générant une réponse inflammatoire systémique (SIRS) dont on sait qu'ils ont survécu, notamment de patients présentant un SEPSIS dont on sait qu'ils ont survécu, et de préférence de patients en état de choc septique dont on sait qu'ils ont survécu. Dans ce cas, et selon une variante particulièrement préférée, l'échantillon de référence est Issu d'un ou de patients en état de choc septique dont on sait qu'il(s) a(ont) survécu.

Pour obtenir la deuxième valeur de référence en particulier, l'échantillon de référence est de préférence un échantillon biologique issu du patient chez qui on souhaite évaluer le risque de mortalité et dont est issu l'échantillon à tester, mais obtenu à partir d'un prélèvement antérieur dans le temps par rapport à celui de l'échantillon à tester. De préférence, la deuxième valeur de référence est obtenue à partir d'un échantillon biologique qui est directement antérieur par rapport à l'échantillon à tester, c'est-à-dire celui qui précède l'échantillon à tester dans l'ordre des prélèvements effectués chez le patient.

Selon un mode de réalisation particulier, le procédé selon l'invention comprend la mesure de l'expression de sCD127 combinée à l'estimation de l'un au moins des scores de sévérité SOFA et/ou SAPSII pour évaluer le risque de mortalité du patient ayant subi une agression ou une infection générant une réponse inflammatoire systémique (SIRS), et en particulier d'un patient qui est en état de choc septique. Dans ce mode de réalisation, le score SOFA est de préférence calculé comme décrit par J.L Vincent et Coll., Intensive Care Med., 1996;22:707-710, et/ou le score SAPSII est de préférence calculé comme décrit par Le Gall J.R. et al., JAMA, 1993;270:2957-63.

Au sens de la présente invention, le terme « mesurer l'expression » s'entend être une mesure *in vitro* ou *ex vivo.* Par ailleurs, ce terme entend désigner la détection et la quantification de sCD127, de préférence au niveau protéique.

A cet effet, toute méthode de détection et/ou de quantification bien connue de l'homme du métier peut être utilisée pour la mise en oeuvre de l'invention, que ce soit en rapport avec la détermination de la présence et/ou la mesure de l'expression de la protéine sCD127. A titre d'exemple de méthode de mesure de l'expression de la protéine sCD127, on peut notamment citer celle décrite par Crawley et al, Journal of Immunology, 2010, 184, 4679-4687.

En particulier, la mesure du niveau d'expression de sCD127 est réalisée à l'aide d'outils ou de réactifs qui sont spécifiques de sCD127 et qui permettent, directement ou indirectement, de déterminer sa présence et/ou de quantifier son niveau d'expression.

Parmi les outils ou réactifs capable(s) de détecter et/ou quantifier sCD127, on peut notamment citer des anticorps spécifiques, polyclonaux ou monoclonaux, de préférence monoclonaux, ou des fragments ou dérivés de ceux-ci, par exemple des fragments Fab, F(ab)'2, Sv, scFv, ou des analogues d'anticorps, en particulier des protéines d'affinité aux propriétés compétitives (les nanofitines™).

Parmi ces outils ou réactifs, on préfère notamment ceux qui sont spécifiques de la forme soluble du récepteur de l'IL-7, c'est-à-dire qui ne reconnaissent pas CD127 qui est la forme cellulaire/membranaire non soluble de ce récepteur. On peut néanmoins utiliser des outils ou réactifs qui reconnaissent à la fois la forme soluble ou sCD127 et la forme cellulaire du récepteur de l'IL-7 ou CD127, tant qu'il est possible de distinguer ces deux formes par un autre moyen, comme par exemple la nature de l'échantillon analysé (par exemple, plasma ou sérum *versus* échantillon biologique contenant des cellules ou sang total).

Lorsque la détection et/ou la quantification de sCD127 est réalisée au niveau protéique, les techniques standards telles que le Western-Blot, ELISA, RIA, IRMA, FIA, CLIA, ECL, cytométrie de flux ou immunocytologie peuvent être utilisées.

De manière particulièrement avantageuse, l'expression de sCD127 est mesurée au niveau protéique, et de préférence à l'aide d'une technique ELISA.

Selon l'invention, et en particulier dans ce mode de réalisation particulier, le niveau d'expression de sCD127 est de préférence mesuré à l'aide d'anticorps anti-sCD127, monoclonaux ou polyclonaux, et notamment d'anticorps monoclonaux anti-sCD127. A titre d'exemple, on peut notamment citer les anticorps monoclonaux anti-CD127 humain R34.34 commercialisés par la société Beckman Coulter® ou les anticorps polyclonaux anti-CD127 commercialisés par la société R&D Systems®.

Toutes les indications et préférences mentionnées ci-dessus s'agissant de la mesure de l'expression de sCD127 s'appliquent indifféremment que ce soit pour la mesure de cette expression dans l'échantillon à tester et dans l'échantillon de référence.

Selon un deuxième aspect, la présente invention a également pour objet l'utilisation de la mesure, *in vitro* ou *ex vivo,* de l'expression de sCD127 pour évaluer le risque de mortalité d'un patient ayant subi une agression, telle que chirurgie, brûlure, traumatisme..., ou une infection générant une réponse inflammatoire systémique ou SIRS, en particulier d'un patient présentant un SEPSIS, notamment un SEPSIS sévère.

De manière préférée, cette utilisation est particulièrement avantageuse pour évaluer le risque de mortalité d'un patient qui est en choc septique.

Par ailleurs, dans le cadre de l'utilisation selon l'invention, l'expression de sCD127 est de préférence mesurée au niveau protéique, et en particulier à l'aide d'une technique ELISA.

En particulier, l'expression de sCD127 peut être mesurée à l'aide d'anticorps anti-sCD127, monoclonaux ou polyclonaux, et de préférence d'anticorps monoclonaux anti-sCD127. Les anticorps cités ci-dessus peuvent également être utilisés pour ce deuxième aspect de l'invention.

Plus largement, tous les modes de réalisation préférés qui sont mentionnés ci-dessus concernant le procédé et leurs combinaisons constituent également des modes de réalisation préférés s'agissant de l'utilisation. Plus particulièrement, l'utilisation selon l'invention peut notamment comprendre la mesure de l'expression de sCD127 combinée à l'estimation de l'un au moins des scores de sévérité SOFA et/ou SAPSII pour évaluer le risque de mortalité du patient ayant subi une agression ou une infection générant une réponse inflammatoire systémique (SIRS), et en particulier d'un patient qui est en état de choc septique.

Selon un troisième aspect, la présente invention a aussi pour objet un kit pour la mesure, *in vitro* ou *ex vivo,* de l'expression de sCD127 dans un échantillon biologique, comprenant :
- des outils ou réactifs spécifiques permettant de mesurer l'expression de sCD127 dans ledit échantillon biologique, et
- un échantillon contrôle positif qui est un échantillon calibré pour contenir la quantité de sCD127 qui correspond à la quantité moyenne mesurée dans un pool d'échantillons de patients dont on sait qu'ils ont survécu, et/ou un échantillon contrôle négatif qui est un échantillon calibré pour contenir la quantité de sCD127 qui correspond à la quantité moyenne mesurée dans un pool d'échantillons de patients dont on sait qu'ils n'ont pas survécu.

Ainsi, le kit selon l'invention comprend des outils ou réactifs spécifiques permettant de mesurer l'expression de sCD127 dans ledit échantillon biologique, et au moins un échantillon contrôle.

Le kit selon l'invention permet en particulier d'évaluer le risque de mortalité chez un patient hospitalisé ayant subi une agression, telle que chirurgie, brûlure, traumatisme..., ou une infection générant une réponse inflammatoire systémique (SIRS), et notamment chez un patient qui est en état de choc septique.

De préférence, les outils ou réactifs spécifiques permettant de mesurer l'expression de sCD127 dans un échantillon biologique qui sont présents dans le kit de l'invention permettent de détecter et/ou de quantifier l'expression de sCD127, et de préférence au niveau protéique.

Selon un mode de réalisation particulièrement préféré, le kit de l'invention contient des anticorps anti-sCD127, monoclonaux ou polyclonaux, et en particulier des anticorps monoclonaux, en tant qu'outils ou réactifs spécifiques permettant de mesurer l'expression de sCD127 dans ledit échantillon biologique.

Un autre échantillon contrôle positif peut également être un échantillon biologique issu d'au moins un patient dont on sait qu'il a survécu. De même, un autre échantillon contrôle négatif peut également être un échantillon biologique issu d'au moins un patient dont on sait qu'il n'a pas survécu. Que ce soit pour un contrôle positif ou négatif, ce type d'échantillon contrôle est notamment issu de plusieurs patients ayant subi une agression, telle que chirurgie, brulûre, traumatisme..., ou une infection générant une réponse inflammatoire systémique (SIRS), notamment de plusieurs patients présentant un SEPSIS, et de préférence de plusieurs patients en état de choc septique.

De manière préférée, le kit comprend à la fois un échantillon contrôle positif et un échantillon contrôle négatif, et en particulier choisis chacun parmi les échantillons calibrés tels que définis ci-dessus.

L'invention couvre également l'utilisation d'un kit selon l'invention pour réaliser le procédé de l'invention, et en particulier pour évaluer le risque de mortalité d'un patient ayant subi une agression, telle que chirurgie, brûlure, traumatisme..., ou une infection générant une réponse inflammatoire systémique (SIRS), en particulier chez un patient présentant un SEPSIS, notamment un SEPSIS sévère. De préférence, l'utilisation du kit selon l'invention permet d'évaluer le risque de mortalité chez un patient qui est en état de choc septique.

Tous les modes de réalisation préférés qui sont mentionnés ci-dessus concernant le procédé et leurs combinaisons constituent également des modes de réalisation préférés s'agissant du kit selon l'invention et de son utilisation.

Diverses autres caractéristiques ressortent de la description faite ci-dessous en référence aux figures annexées qui montrent, à titre d'exemples non limitatifs, des formes de réalisation de l'objet de l'invention et dans lesquelles :
- la **Figure 1** représente la courbe ROC de la concentration de sCD127 plasmatique mesurée à J1-2, du score SAPSII et du score SOFA chez 70 patients après choc septique.
- les **Figures 2A, 2B** **et** **2C** représentent les courbes de survie de sCD127 pour 70 patients à J1-2 (**A**) et à J3-4 (**B**) et du score SAPSII (**C**) après choc septique.
- les **Figures 3A et 3B** représentent les courbes de survie de la combinaison de sCD127 pour 70 patients à J1-2 (**A**) et à J3-4 (**B**) après choc septique et du score SAPSII.

### METHODES

### Echantillons biologiques

Des échantillons de plasma ont été prélevés sur 70 patients en choc septique aux jours 1-2 (J1-2) et 3-4 (J3-4) après choc septique, puis ont été stockés (cohorte rétrospective). Des échantillons plasmatiques ont également été prélevés chez 41 sujets sains volontaires.

A 28 jours après admission en réanimation pour choc septique, 14 patients n'ont pas survécu (« NS ») soit 20 %, alors que 56 patients ont survécu (« S ») sur les 70 patients.

### Dosage de la forme soluble du récepteur de l'IL-7 (sCD127) par ELISA

### « Coating »

Un tampon de « coating » a été préparé pour contenir 0,8 g Na₂CO₃, 1,4 g NaHCO₃, 0,1 g NaN₃ dans 500 ml d'eau (pH 9.6).

100 µL d'anticorps (Ac) de capture (anticorps monoclonal de souris anti-CD127 humain R34.34, Beckman Coulter®) dilués dans du tampon de « coating » ont été déposés par puits dans une plaque ([Ac] = 8 µg/mL). La plaque a ensuite été recouverte pour être incubée à 4°C pendant une nuit.

Le contenu des puits a ensuite été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### « Blocking »

La fixation non spécifique a été bloquée à l'aide de 150 µL de tampon de blocage par puits (10% sérum de bovin foetal (FBS)/PBS-Tween₂₀ 0,05%), puis la plaque a été incubée pendant 1h à 37°C.

De nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### Échantillons et contrôles

Une gamme étalon a été réalisée avec recombinant human IL-7Rα / CD127 Fc chimera (R&D Systems - Catalog Number: 306-IR) dilué dans du tampon de dilution PBS 5 % FCS, comme décrit dans le **Tableau 1** ci-dessous et conformément à C. Janot-Sardet et al. Journal of Immunological Methods, 2010, 28, 115-123.

**Tableau 1**

| | rh IL-7Rα / CD127 Fc chimera | | | | | | | |
|---|---|---|---|---|---|---|---|---|
| [c] (ng/mL) | 500 | 250 | 125 | 62.5 | 31.25 | 15.7 | 7.85 | 0 |
| Diluant (µL) | 0 | 100 | 100 | 100 | 100 | 100 | 100 | 100 |
| Solution à 500 ng/mL (µL) | 100 | 100 | Dilutions successives | | | | | 0 |

100 µL d'échantillon ou de contrôle (solution de CD127 Fc chimera reconstitué extemporanément et aliquoté à des concentrations de 60ng/ml et 10ng/ml) ont été ajoutés dans chaque puits, puis la plaque a été incubée pendant 1h à 37°C.

De nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### Anticorps de détection

100 µL d'anticorps de détection (anticorps de chèvre polyclonal anti-CD127 biotinylé reconstitués avec 1 mL de TBS-BSA 1%, R&D Systems®) dilués dans du PBS/5 %FBS ont été ajoutés dans chaque puits ([Ac] = 200 ng/mL), puis la plaque a été incubée pendant 1h à 37°C.

A nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### Révélation

100 µL de Streptavidin-HRP ont été ajoutés dans chaque puits ([Streptavidin-HRP] = 8 µL/mL). La plaque a ensuite été recouverte pour être incubée pendant 30 min à température ambiante.

A nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon. A cette étape de lavage, les puits ont été imbibés avec le tampon de lavage 1 à 2 min avant aspiration.

Les deux flacons de la solution de substrat calorimétrique TMB (3,3',5,5'-tetramethylbenzidine, bioMérieux #XX7LF1UC) ont été mélangés volume à volume et 100 µL de cette solution de substrat ont été déposés dans chaque puits. La plaque a ensuite été recouverte pour être incubée pendant 30 min à température ambiante.

Enfin, la lecture de la plaque a été effectuée par mesure de l'absorbance à 450 nm.

### Analyse multivariée

Un modèle de Cox a permis d'estimer le « *Hazard Ratio* » et son intervalle de confiance à 95 % (95%CI) et sa significativité. Les scores SAPSII et SOFA ont été inclus dans le modèle sous forme de variables continues en assumant une relation linéaire entre leurs valeurs et le risque de décès (survivants vs. non survivants). Les analyses statistiques ont été réalisées grâce aux logiciels SPSS (version 17.0, SPSS, Chicago, IL) et GraphPad Prism (version 5.03, GraphPad Software, La Jolla, CA). Des valeurs de p inférieures à 0,05 ont été considérées comme significatives.

### Analyse de comparaison de survie (Test du « Log Rank »)

Les courbes ROC (*Receiver Operating Curves*) ont été générées à l'aide du logiciel cité ci-dessus et la concentration optimale ou seuil optimum de sCD127 permettant d'obtenir les meilleurs sensibilités et spécificités a été définie, grâce à l'index de Youden qui combine les paramètres de sensibilité et spécificité pour des valeurs incrémentées du marqueur considéré. La concentration optimale du marqueur qui donne l'index de Youden le plus élevé, c'est-à-dire une sensibilité et une spécificité optimales, a donc été déterminée. Les courbes de survie de Kaplan-Meier ont été obtenues après stratification des patients sur la base de cette valeur optimale. La différence de survie entre les groupes a été évaluée par le test du « *Log Rank* » et le « *Hazard Ratio* » (et son intervalle de confiance à 95%) calculé sur la base des pentes de ces courbes de survie.

### RESULTATS

### Dosage de la forme soluble du récepteur de l'IL-7 (sCD127)

La concentration de sCD127 plasmatique a été mesurée comme décrit ci-dessus dans les échantillons de plasma de 70 patients en choc septique et les scores de sévérité SOFA et SAPSII ont été évalués chez ces patients sur la base des données cliniques et physiologiques disponibles au cours des 24 premières heures d'hospitalisation.

Les mêmes mesures ont également été effectuées sur les échantillons issus de 41 sujets sains volontaires (HV).

Les résultats sont regroupés dans les tableaux **2 à 4** qui suivent :

### Capacité de sCD127 à prédire le décès des patients en choc septique

La capacité prédictive de la mesure de la concentration du sCD127 plasmatique a été étudiée au regard de l'événement à étudier, à savoir la mortalité. Celle de deux scores de référence SAPSII et SOFA a également été étudiée par rapport à ce même événement.

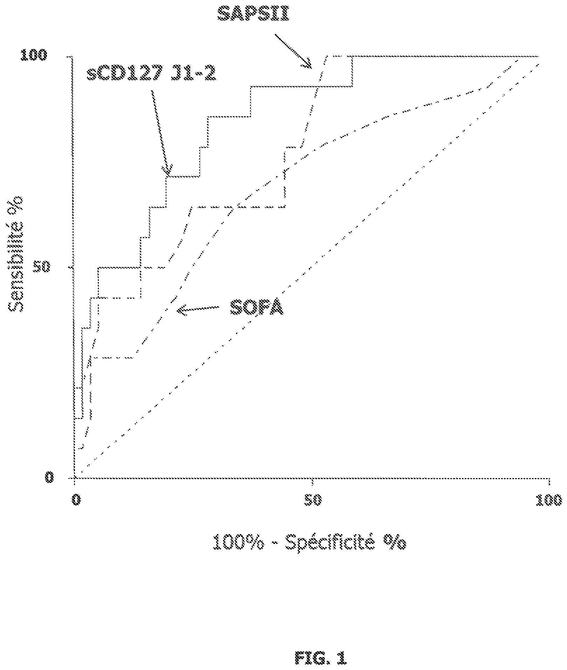

Les résultats sont représentés sur la **Figure 1** sous la forme d'une courbe ROC (*Receiving Operating Curve*) du sCD127 mesuré à J1-2 et des scores de sévérité SAPSII et SOFA chez les 70 patients en choc septique.

L'évaluation des aires sous la courbe, reprises dans le **Tableau 2** ci-dessous, permet de comparer les performances prédictives des scores connus SOFA et SAPSII avec celle de la mesure du sCD127 plasmatique.

**Tableau 2**

| | Aire sous la courbe (AUC) | Valeur de p |
|---|---|---|
| sCD127 mesuré à J1-2 | 0,846 | P<0,001 |
| sCD127 mesuré à J3-4 | 0,774 | P=0,002 |
| SOFA à J1 | 0,692 | P=0,027 |
| SAPSII à J1 | 0,770 | P=0,002 |

Pour les valeurs de sCD127 mesurées à J1-2, le seuil optimum a été défini à 44,45 ng/ml grâce à l'index de Youden, ce qui amène une sensibilité de 86% ; une spécificité de 71%, une valeur prédictive positive (probabilité que le patient soit décédé si le test est au dessus du seuil) de 43% et une valeur prédictive négative (probabilité que le patient soit survivant lorsque le test est en dessous du seuil) de 95 %.

De même, pour les valeurs de sCD127 mesurées à J3-4, le seuil optimum a été défini à 48,10 ng/ml, ce qui amène une sensibilité de 71% ; une spécificité de 86%, une valeur prédictive positive de 56 % et une valeur prédictive négative de 92 %.

Par comparaison, par le score SOFA mesuré à J1, la sensibilité est de 64,3% et la spécificité est de 66,1% avec une valeur prédictive positive de 32,1% et une valeur prédictive négative de 88,1%.

Par le score SAPSII mesuré à J1, la sensibilité est de 71,4% et la spécificité est de 55,4% avec une valeur prédictive positive de 28,6% et une valeur prédictive négative de 88,6%.

Ces résultats montrent donc que la mesure à J1-2 de la concentration de sCD127 dans le plasma des patients présente une capacité de prédiction du décès post-choc septique meilleure que les scores classiquement utilisés SOFA et SAPSII (sur la base des aires sous les courbes ROC).

### Analyse multivariée

Afin d'évaluer l'indépendance du lien entre la concentration plasmatique du sCD127 et le risque de décès par rapport aux facteurs de risques connus des patients en choc septique (sévérité initiale et nombre de défaillances d'organes évalués par les scores SAPSII et SOFA), des analyses de régression logistique uni et multivariées ont été effectuées comme décrit ci-dessus.

Une analyse muitivariée entre les scores SOFA, SAPSII et la mesure de sCD127 obtenus à J1-2 et J3-4 a été conduite. Les résultats, qui sont regroupés dans les **Tableaux 3 et 4** suivants, montrent que la mesure de sCD127 est un marqueur qui est tout à fait indépendant des deux facteurs de risque connus SOFA et SAPSII pour la prédiction de la mortalité post-choc septique.

**Tableau 3**

| | *Valeur de p* | *« Hazard Ratio »* | Intervalle de confiance à 95% |
|---|---|---|---|
| **sCD127 J1-2** | **0,004** | 8,153 | 1,927-34,503 |
| **SOFA** | 0,872 | 1,125 | 0268-4718 |
| **SAPSII** | 0,062 | 3,887 | 0,936-16,149 |

**Tableau 4**

| | *Valeur de p* | *« Hazard Ratio »* | Intervalle de confiance à 95% |
|---|---|---|---|
| **sCD127 J3-4** | **0,011** | 6,748 | 1,55-29,374 |
| **SOFA** | 0,599 | 1,436 | 0,373-5,526 |
| **SAPSII** | 0,06 | 3,787 | 0,947-15,141 |

### Courbes de survie selon Kaplan-Meier

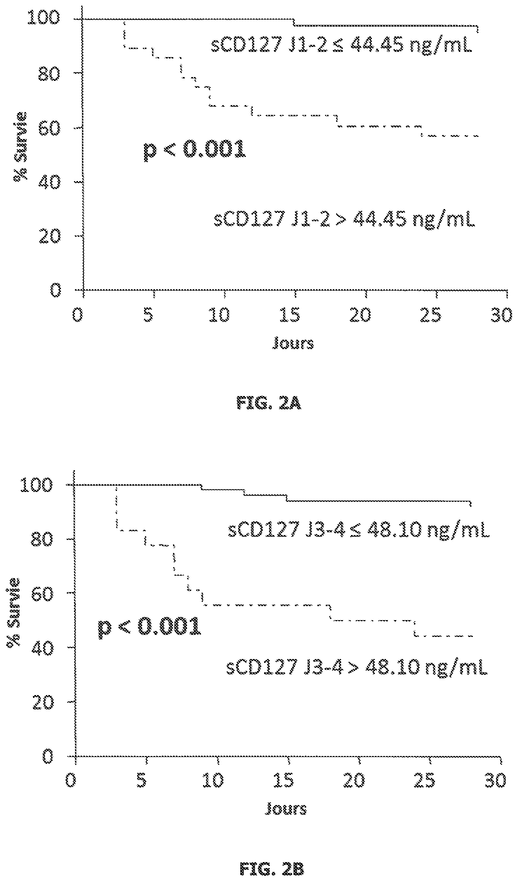

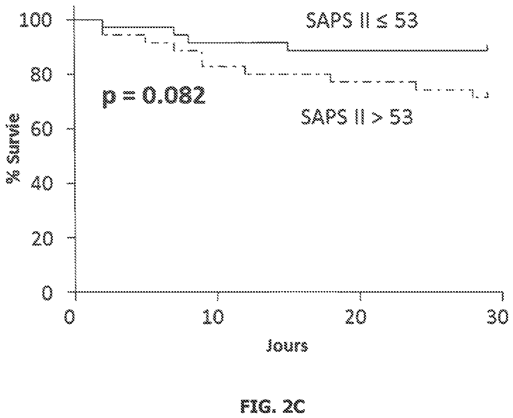

Des courbes de survie ont été établies sur la base des valeurs de seuil optimum déterminées à l'aide des courbes ROC et de l'indice de Youden de sCD127 à J1-2 et à J3-4. La courbe de survie après stratification des patients sur la base du score SAPSII a également été générée (seuil égal à 53). Les résultats sont représentés sur les **Figures 2A, 2B****,** et **2C****.**

Que ce soit à J1-2 (**fig. 2A**) ou J3-4 (**fig. 2B**), ces résultats montrent une différence significative entre les deux courbes de survie tracées en fonction du seuil optimum de sCD127 (J1-2 (**fig. 2A**) : « *Hazard ratio »* = 10,22 ; [95%Cl] : 3,36-31,13 ; p<0,001, Test du « *Log Rank* » ; J3-4 (**fig. 2B**) : « *Hazard ratio* » = 28,50; [95%Cl] : 7,519-108,0) ; p<0,001, Test du « *Log rank* ».

A l'inverse, après stratification sur la base du score SAPSII, aucune différence significative de mortalité n'a été mise en évidence (**fig. 2C** : p = 0.082, Test du « *Log Rank* », *« Hazard ratio »* = 2.55, [95%CI]: 0.887-7.309).

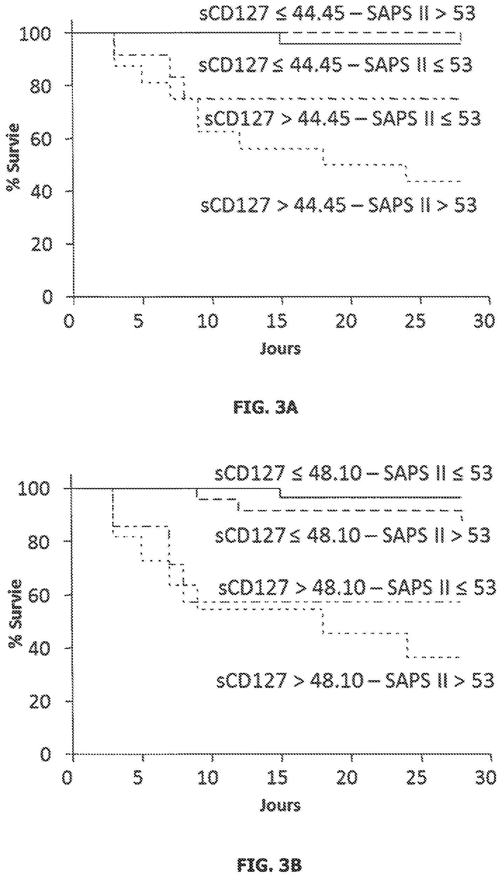

Enfin, la capacité prédictive sur le risque de décès de la combinaison entre une valeur augmentée de sCD127 et un score SAPSII augmenté a été évaluée comme décrit ci-dessus, après stratification des patients sur la base des valeurs de seuil optimum déterminées à l'aide des courbes ROC et de l'indice de Youden de sCD127 à J1-2 et à J3-4 et du seuil optimum de SAPSII (seuil égal à 53). Les courbes de survie correspondantes ont donc également été générées, et les résultats sont représentés sur les **Figures 3A** et **3B** respectivement

Que ce soit à J1-2 ou J3-4, ces résultats montrent que les patients qui ont les plus faibles concentrations de sCD127 et le plus faible score SAPSII ont une bien meilleure chance de survie, comparé à ceux qui présentent les plus fortes concentrations de sCD127 et le plus fort score SAPSII :
J1-2 (**fig. 3A**) : p<0,001, Test du *Log Rank, Hazard ratio* = 12,98 ; [95%Cl] : 3,453-48,83.
J3-4 (**fig. 3B**) : p<0,001, Test du *Log Rank, Hazard ratio* = 50,94; [95%Cl] : 9,352-277,4).

Ces résultats démontrent que la mesure combinée de sCD127 et de l'un au moins des scores SAPSII ou SOFA permet d'augmenter la force prédictive eu égard à la mortalité chez les patients hospitalisés en réanimation.

En conséquence, l'ensemble de ces résultats démontrent que la mesure de l'expression de sCD127 dans le plasma est un marqueur immunologique très utile et fiable pour évaluer le risque de mortalité d'un patient admis dans un service de réanimation, et notamment d'un patient en état de choc septique. De plus, ce paramètre pourrait être combiné aux scores cliniques habituels pour améliorer la capacité prédictive du risque de décès de ces scores.

L'invention n'est pas limitée aux exemples décrits et représentés car diverses modifications peuvent y être apportées sans sortir de son cadre.

## Revendications

1. Procédé *in vitro* ou *ex vivo* d'évaluation du risque de mortalité chez un patient ayant subi une agression ou une infection générant une réponse inflammatoire systémique, comprenant les étapes suivantes :
- mesurer l'expression de sCD127 dans un échantillon biologique issu dudit patient, également nommé échantillon à tester,
- conclure à un risque accru de mortalité après comparaison de l'expression de sCD127 par rapport à une valeur de référence.

2. Procédé selon la revendication 1, **caractérisé en ce que** l'échantillon biologique est issu d'un patient présentant un SEPSIS, notamment un SEPSIS sévère.

3. Procédé selon la revendication 2, **caractérisé en ce que** l'échantillon biologique est issu d'un patient en état de choc septique.

4. Procédé selon l'une quelconque des revendications 1 à 3, **caractérisé en ce qu'**il est conclu à un risque accru de mortalité chez ledit patient, lorsqu'une surexpression de sCD127 est mise en évidence dans l'échantillon à tester par rapport à une première valeur de référence qui correspond à une valeur moyenne du niveau d'expression de sCD127 qui est mesuré sur un pool d'échantillons issu de patients ayant subi une agression ou une infection générant une réponse inflammatoire systémique ou SIRS dont on sait qu'ils ont survécu.

5. Procédé selon les revendications 3 et 4, **caractérisé en ce que** la première valeur de référence correspond à une valeur moyenne du niveau d'expression de sCD127 qui est mesuré sur un pool d'échantillons issu de patients en état de choc septique dont on sait qu'ils ont survécu.

6. Procédé selon la revendication 5, **caractérisé en ce que** la mesure de l'expression de sCD127 dans l'échantillon à tester et le cas échéant dans l'échantillon biologique utilisé pour obtenir la première valeur de référence est réalisée dans les 2 jours ou dans le jour après le choc septique, ou encore à 2 jours ou à 1 jour après le choc septique.

7. Procédé selon l'une quelconque des revendications 1 à 3, **caractérisé en ce qu'**il est conclu à un risque accru de mortalité chez ledit patient, lorsque l'expression de sCD127 qui est mesurée dans l'échantillon à tester n'est pas significativement diminuée par rapport à une deuxième valeur de référence qui correspond au niveau d'expression de sCD127 mesuré dans un échantillon biologique issu du même dit patient lors d'un prélèvement antérieur.

8. Procédé selon la revendication 3, ou selon la revendication 7, **caractérisé en ce que** la mesure de l'expression de sCD127 dans l'échantillon à tester est réalisée à environ ou 4 jours (J4) après le choc septique, en particulier environ 3 jours ou à 3 jours (J3) après le choc septique, et de manière encore préférée environ 2 jours ou à 2 jours (J2) après le choc septique.

9. Procédé selon l'une quelconque des revendications 3, 7 et 8, **caractérisé en ce que** le prélèvement antérieur est effectué dans les ou à 48h après le choc septique et au moins 24h avant celui de l'échantillon à tester.

10. Procédé selon l'une quelconque des revendications précédentes, **caractérisé en ce que** la mesure de l'expression de sCD127 est combinée à l'estimation de l'un au moins des scores de sévérité SOFA et/ou SAPSII pour évaluer le risque de mortalité pour ledit patient.

11. Procédé selon l'une quelconque des revendications précédentes, **caractérisé en ce que** l'expression de sCD127 est mesurée à l'aide d'anticorps anti-sCD127, et notamment d'anticorps monoclonaux anti-sCD127.

12. Utilisation de la mesure, *in vitro* ou *ex vivo,* de l'expression de sCD127 pour évaluer le risque de mortalité d'un patient ayant subi une agression ou une infection générant une réponse inflammatoire systémique.

13. Utilisation selon la revendication 12, **caractérisée en ce que** ledit patient est un patient qui est en état de choc septique.

14. Utilisation selon l'une quelconque des revendications 12 ou 13, **caractérisé en ce que** l'expression de sCD127 est mesurée à l'aide d'anticorps anti-sCD127, de préférence d'anticorps monoclonaux anti-sCD127.

15. Kit pour la mesure, *in vitro* ou *ex vivo,* de l'expression de sCD127 dans un échantillon biologique, comprenant :
- des outils ou réactifs spécifiques permettant de mesurer l'expression de sCD127 dans ledit échantillon biologique, et
- un échantillon contrôle positif qui est un échantillon calibré pour contenir la quantité de sCD127 qui correspond à la quantité moyenne mesurée dans un pool d'échantillons de patients ayant subi une agression ou une infection générant une réponse inflammatoire systémique, dont on sait qu'ils ont survécu, et/ou un échantillon contrôle négatif qui est un échantillon calibré pour contenir la quantité de sCD127 qui correspond à la quantité moyenne mesurée dans un pool d'échantillons de patients ayant subi une agression ou une infection générant une réponse inflammatoire systémique, dont on sait qu'ils n'ont pas survécu.

16. Kit selon la revendication 15, **caractérisé en ce qu'**il contient des anticorps anti-sCD127 en tant qu'outils ou réactifs spécifiques permettant de mesurer l'expression de sCD127 dans ledit échantillon biologique.

## Patentansprüche

1. *In-vitro-* oder *Ex-vivo*-Verfahren zur Bewertung des Sterblichkeitsrisikos bei einem Patienten, der einen Angriff oder eine Infektion erlitten hat, die eine systemische Entzündungsreaktion hervorruft, umfassend die folgenden Schritte:
- Messen der Expression von sCD127 in einer biologischen Probe, die von dem Patienten stammt, die auch zu testende Probe genannt wird,
- Schließen auf ein erhöhtes Sterblichkeitsrisiko nach Vergleichen der Expression von sCD127 mit einem Referenzwert.

2. Verfahren nach Anspruch 1, **dadurch gekennzeichnet, dass** die biologische Probe von einem Patienten stammt, der eine SEPSIS, insbesondere eine schwere SEPSIS aufweist.

3. Verfahren nach Anspruch 2, **dadurch gekennzeichnet, dass** die biologische Probe von einem Patienten in septischem Schock stammt.

4. Verfahren nach einem der Ansprüche 1 bis 3, **dadurch gekennzeichnet, dass** auf ein erhöhtes Sterblichkeitsrisiko bei dem Patienten geschlossen wird, wenn eine Überexpression von sCD127 in der zu testenden Probe gegenüber einem ersten Referenzwert nachgewiesen wird, der einem Durchschnittswert des Expressionsniveaus von sCD127 entspricht, das an einem Probenpool gemessen wird, der von Patienten stammt, die einen Angriff oder eine Infektion erlitten haben, die eine systemische Entzündungsreaktion oder SIRS hervorruft, von denen bekannt ist, dass sie überlebt haben.

5. Verfahren nach Anspruch 3 und 4, **dadurch gekennzeichnet, dass** der erste Referenzwert einem Durchschnittswert des Expressionsniveaus von sCD127 entspricht, das an einem Probenpool gemessen wird, der von Patienten in septischem Schock stammt, von denen bekannt ist, dass sie überlebt haben.

6. Verfahren nach Anspruch 5, **dadurch gekennzeichnet, dass** die Messung der Expression von sCD127 in der zu testenden Probe und gegebenenfalls in der biologischen Probe, die für den Erhalt des ersten Referenzwertes verwendet wird, innerhalb von 2 Tagen oder am Tag nach dem septischen Schock oder auch 2 Tage oder 1 Tag nach dem septischen Schock durchgeführt wird.

7. Verfahren nach einem der Ansprüche 1 bis 3, **dadurch gekennzeichnet, dass** auf ein erhöhtes Sterblichkeitsrisiko bei dem Patienten geschlossen wird, wenn die Expression von sCD127, die in der zu testenden Probe gemessen wird, gegenüber einem zweiten Referenzwert nicht signifikant abgenommen hat, der dem Expressionsniveau von sCD127 entspricht, das in einer biologischen Probe, welche von dem gleichen Patienten aus einer früheren Entnahme stammt, gemessen wird.

8. Verfahren nach Anspruch 3 oder nach Anspruch 7, **dadurch gekennzeichnet, dass** die Messung der Expression von sCD127 in der zu testenden Probe ungefähr 4 Tage (J4) nach dem septischen Schock, insbesondere ungefähr 3 Tage oder 3 Tage (J3) nach dem septischen Schock und besonders bevorzugt ungefähr 2 Tage oder 2 Tage (J2) nach dem septischen Schock durchgeführt wird.

9. Verfahren nach einem der Ansprüche 3, 7 und 8, **dadurch gekennzeichnet, dass** die frühere Entnahme innerhalb von oder 48 h nach dem septischen Schock und mindestens 24 h vor derjenigen der zu testenden Probe durchgeführt wird.

10. Verfahren nach einem der vorhergehenden Ansprüche, **dadurch gekennzeichnet, dass** die Messung der Expression von sCD127 mit der Schätzung von mindestens einem der Schweregrade von SOFA und/oder SAPSII kombiniert wird, um das Sterblichkeitsrisiko für diesen Patienten zu bewerten.

11. Verfahren nach einem der vorhergehenden Ansprüche, **dadurch gekennzeichnet, dass** die Expression von sCD127 mit Hilfe von Antikörpern Anti-sCD127 und insbesondere von monoklonalen Antikörpern Anti-sCD127 gemessen wird.

12. Verwendung der *In-vitro-* oder *Ex-vivo*-Messung der Expression von sCD127 zur Bewertung des Sterblichkeitsrisikos eines Patienten, der einen Angriff oder eine Infektion erlitten hat, die eine systemische Entzündungsreaktion hervorruft.

13. Verwendung nach Anspruch 12, **dadurch gekennzeichnet, dass** der Patient ein Patient ist, der in septischem Schock ist.

14. Verwendung nach einem der Ansprüche 12 oder 13, **dadurch gekennzeichnet, dass** die Expression von sCD127 mit Hilfe von Antikörpern Anti-sCD127, vorzugsweise von monoklonalen Antikörpern Anti-sCD127 gemessen wird.

15. Kit für die *In-vitro-* oder *Ex-vivo*-Messung der Expression von sCD127 in einer biologischen Probe, umfassend:
- spezifische Instrumente oder Reagenzien, die ermöglichen, die Expression von sCD127 in der biologischen Probe zu messen, und
- eine positive Kontrollprobe, die eine kalibrierte Probe ist, um die Menge an sCD127 zu enthalten, welche der durchschnittlichen Menge entspricht, die in einem Probenpool von Patienten gemessen wird, die einen Angriff oder eine Infektion erlitten haben, die eine systemische Entzündungsreaktion hervorruft, von denen bekannt ist, dass sie überlebt haben, und/oder eine negative Kontrollprobe, die eine kalibrierte Probe ist, um die Menge an sCD127 zu enthalten, welche der durchschnittlichen Menge entspricht, die in einem Probenpool von Patienten gemessen wird, die einen Angriff oder eine Infektion erlitten haben, die eine systemische Entzündungsreaktion hervorruft, von denen bekannt ist, dass sie nicht überlebt haben.

16. Kit nach Anspruch 15, **dadurch gekennzeichnet, dass** es die Antikörper Anti-sCD127 als spezifische Instrumente oder Reagenzien enthält, die ermöglichen, die Expression von sCD127 in der biologischen Probe zu messen.

## Claims

1. A method *in vitro* or *ex vivo* of evaluating the risk of mortality in a patient who has been subjected to an insult or to an infection generating a systemic inflammatory response, the method comprising the following steps:
- measuring the expression of sCD127 in a biological sample obtained from said patient, also known as the test sample; and
- concluding whether there is an increased risk of mortality after comparing the expression of sCD127 with a reference value.

2. A method according to claim 1, **characterized in that** the biological sample is obtained from a patient presenting with a SEPSIS, in particular a severe SEPSIS.

3. A method according to claim 2, **characterized in that** the biological sample is obtained from a patient in a state of septic shock.

4. A method according to any one of claims 1 to 3, **characterized in that** it is concluded that there is an increased risk of mortality in said patient when an overexpression of sCD127 is demonstrated in the test sample compared with a first reference value which corresponds to a mean value for the level of expression of sCD127 that is measured on a pool of samples obtained from patients who have been subjected to an insult or to an infection generating a systemic inflammatory response or SIRS who are known to have survived.

5. A method according to claims 3 and 4, **characterized in that** the first reference value corresponds to a mean value for the level of expression of sCD127 that is measured on a pool of samples obtained from patients in septic shock who are known to have survived.

6. A method according to claim 5, **characterized in that** the expression of sCD127 in the test sample and if appropriate in the biological sample used to obtain the first reference value is measured within 2 days or within the day after the septic shock, or 2 days or 1 day after the septic shock.

7. A method according to any one of claims 1 to 3, **characterized in that** an increased risk of mortality in said patient is concluded when the expression of sCD127 that is measured in the test sample is not significantly reduced compared with a second reference value which corresponds to the level of expression of sCD127 measured in an anteriorly obtained biological sample taken from the same said patient.

8. A method according to claim 3 or according to claim 7, **characterized in that** the expression of sCD127 in the test sample is measured on or about 4 days (D4) after the septic shock, in particular on or about 3 days (D3) after the septic shock, and more preferably on or about 2 days (D2) after the septic shock.

9. A method according to any one of claims 3, 7 and 8, **characterized in that** the anterior sample is taken within 48 h or 48 h after the septic shock and at least 24 h before that of the test sample.

10. A method according to any preceding claim, **characterized in that** the measurement of the expression of sCD127 is combined with estimating at least one of the SOFA and/or SAPSII severity scores in order to evaluate the risk of mortality for said patient.

11. A method according to any preceding claim, **characterized in that** the expression of sCD127 is measured with the aid of anti-sCD127 antibody, in particular monoclonal anti-sCD127 antibody.

12. The use of the *in vitro* or *ex vivo* measurement of the expression of sCD127 to evaluate the risk of mortality in a patient who has been subjected to an insult or to an infection generating a systemic inflammatory response.

13. Use according to claim 12, **characterized in that** said patient is a patient who is in a state of septic shock.

14. Use according to claim 12 or claim 13, **characterized in that** the expression of sCD127 is measured with the aid of anti-sCD127 antibody, preferably monoclonal anti-sCD127 antibody.

15. A kit for *in vitro* or *ex vivo* measurement of the expression of sCD127 in a biological sample, comprising:
- specific tools or reagents for measuring the expression of sCD127 in said biological sample; and
- a positive control sample, which is a sample calibrated to contain the quantity of sCD127 that corresponds to the mean quantity measured in a pool of samples from patients who have been subjected to an insult or to an infection generating a systemic inflammatory response who are known to have survived, and/or a negative control sample, which is a sample calibrated to contain the quantity of sCD127 that corresponds to the mean quantity measured in a pool of samples from patients who have been subjected to an insult or to an infection generating a systemic inflammatory response who are known not to have survived.

16. A kit according to claim 15, **characterized in that** it contains anti-sCD127 antibodies as specific tools or reagents for measuring the expression of sCD127 in said biological sample.
